(19) Europäisches Patentamt
European Patent Office
Office européen des brevets

(11) Veröffentlichungsnummer: **0 073 378**
**B1**

# (12) EUROPÄISCHE PATENTSCHRIFT

(45) Veröffentlichungstag der Patentschrift: **19.06.85**

(51) Int. Cl.⁴: **C 07 D 339/08**, C 09 K 19/34

(21) Anmeldenummer: **82107344.2**

(22) Anmeldetag: **13.08.82**

## (54) 2,5-disubstituierte-1,3-Dithiane, Verfahren zu deren Herstellung und deren Verwendung.

(30) Priorität: **27.08.81 DE 3133884**

(43) Veröffentlichungstag der Anmeldung: **09.03.83 Patentblatt 83/10**

(45) Bekanntmachung des Hinweises auf die Patenterteilung: **19.06.85 Patentblatt 85/25**

(84) Benannte Vertragsstaaten: **CH DE FR GB IT LI NL**

(56) Entgegenhaltungen:
**US - A - 3 666 786**
**US - A - 3 772 334**

**CHEMICAL ABSTRACTS, Band 81, 1974, Seite 450, Nr. 104580c, Columbus, Ohio, USA L. ANGIOLINI et al.: "Conformational analysis of saturated heterocycles. Conformation of 5-aryl groups in 1,3-dithianes"**

(73) Patentinhaber: **BAYER AG, Konzernverwaltung RP Patentabteilung, D-5090 Leverkusen 1 Bayerwerk (DE)**

(72) Erfinder: **Öller, Manfred, Dr., Breslauer Strasse 31, D-4150 Krefeld (DE)**

EP 0 073 378 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue 2,5-disubstituierte-1,3-Dithiane, ein Verfahren zu deren Herstellung sowie deren Verwendung in optoelektronischen Bauelementen.

2,5-disubstituierte-1,3-Dithiane sind im Chemical Abstracts, Band 81, 1974, Seite 450, Nr. 104 580c beschrieben.

Es ist bekannt, daß nematische flüssige Kristalle aufgrund ihrer dielektrischen Anisotropie verschiedene elektrooptische Effekte zeigen, die zur Herstellung von optoelektronischen Bauelementen genutzt werden können (vgl. z. B. G. Meier, E. Sackmann, J. G. Grabmaier, Applications of Liquid Crystals, Berlin-Heidelberg-New York, Springer-Verlag 1975). Nematische flüssige Kristalle mit negativer dielektrischer Anisotropie zeigen den dynamischen Streueffekt, der die Konstruktion von optoelektronischen Bauelementen ohne die Verwendung von Polarisatoren erlaubt. Von besonderem Interesse sind Bauelemente auf der Basis künstlich verdrillter nematischer Schichten von Substanzen mit positiver dielektrischer Anisotropie, wobei die Deformation der Verdrillung unter Einwirkung eines elektrischen Feldes zu Hell-Dunkel-Effekten führt.

Es gibt aber zur Zeit weder eine reine Verbindung noch Gemische mehrerer Substanzen, welche alle Anforderungen erfüllen, die an eine Substanz für optoelektronische Bauelemente gestellt werden, insbesondere niedrige Schmelz- und hohe Klärpunkte, Farblosigkeit und Beständigkeit unter Einwirkung von Licht, Chemikalien und thermischer Belastung.

Es wurde nun eine neue, zur Verwendung in Flüssigkristall-Anzeigen geeignete Substanzklasse, nämlich 2,5-disubstituierte-1,3-Dithiane, gefunden, die günstige Eigenschaften hinsichtlich des Schmelz- und Klärpunkts, der Farblosigkeit sowie der Beständigkeit unter Einwirkung von Licht, Chemikalien und thermischer Belastung aufweist.

Die Erfindung betrifft daher neue 2,5-disubstituierte-1,3-Dithiane der Formel

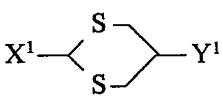 (I)

in der

$X^1$ = 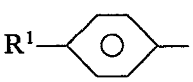 und $Y^1$ = $R^2$ oder

$X^1$ = $R^3$ und $Y^1$ = 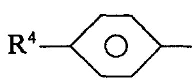 oder

$X^1$ = 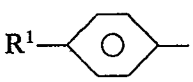 und $Y^1$ = 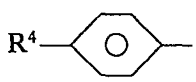

bedeuten und

$R^1$ = $C_nH_{2n+1}$, $C_nH_{2n+1}O$, $C_nH_{2n+1}S$, $C_nH_{2n+1}COO$, $C_nH_{2n+1}OCOO$, $(C_2H_5)_2N$, $(CH_3)_2N$, F, Cl, Br, $NO_2$, CN,

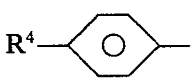 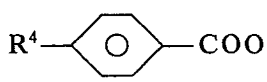 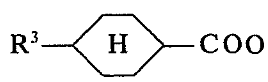

$R^2$ = $C_nH_{2n+1}$, $OR^3$, $SR^3$, $R^3COO$, $R^3OCO$, 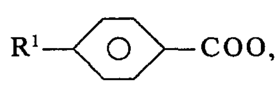, 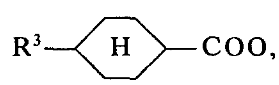, CN,

$R^3$ = $C_nH_{2n+1}$ und

$R^4$ = $C_nH_{2n+1}$, $C_nH_{2n+1}O$, F, Cl, Br, $NO_2$, CN,

mit n = 1 bis 12 sein kann.

Weiterhin betrifft die Erfindung die Herstellung der neuen 2,5-disubstituierten-1,3-Dithiane sowie deren Verwendung in optoelektronischen Bauelementen.

Bevorzugt sind die Verbindungen der Formel

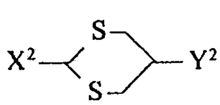 (II)

in der

$X^2$ = 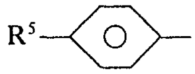 und $Y^2 = R^6$ oder

$X^2 = R^7$ und $Y^2$ = 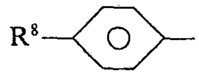 oder

$X^2$ = 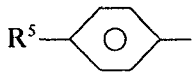 und $Y^2$ = 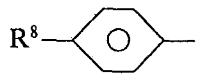

bedeuten und

$R^5 = C_nH_{2n+1}, C_nH_{2n+1}O, C_nH_{2n+1}S, C_nH_{2n+1}COO$, F, CN,

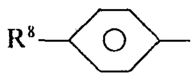 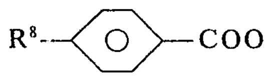 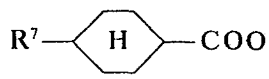

$R^6 = C_nH_{2n+1}, OR^7, SR^7,$
$R^7 = C_nH_{2n+1}$ und
$R^8 = C_nH_{2n+1}, C_nH_{2n+1}O$, F, CN,

mit n = 3 bis 9 sein kann.

Besonders bevorzugt sind die Verbindungen der Formel

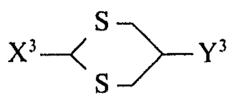 (III)

in der

$X^3$ = 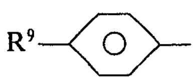 und $Y^3 = R^{10}$

bedeuten und

$R^9 = C_nH_{2n+1}, C_nH_{2n+1}O, C_nH_{2n+1}S, C_nH_{2n+1}COO$, F, CN,

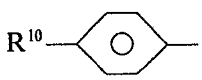 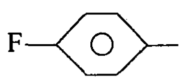 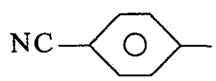 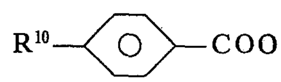

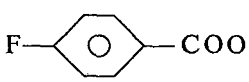 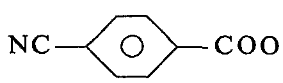 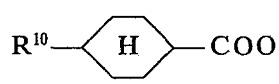

und

$R^{10} = C_nH_{2n+1}, OC_nH_{2n+1},$

mit n = 3 bis 9 sein kann.

Als 2,5-disubstituierte-1,3-Dithiane seien beispielsweise genannt:

2-(4-Propylphenyl)-5-heptyl-1,3-dithian
2-(4-Pentylphenyl)-5-heptyl-1,3-dithian
2-(4-Heptylphenyl)-5-heptyl-1,3-dithian
2-(4-Cyanophenyl)-5-butyl-1,3-dithian
2-(4-Butyloxyphenyl)-5-propyl-1,3-dithian
2-Pentyl-5-(4-heptylphenyl)-1,3-dithian
2-Heptyl-5-(4-heptylphenyl)-1,3-dithian
2-Heptyl-5-(4-heptyloxyphenyl)-1,3-dithian
2-Heptyl-5-(4-cyanophenyl)-1,3-dithian
2-Heptyl-5-(4-fluorophenyl)-1,3-dithian
2-(4-Propylphenyl)-5-(4-propylphenyl)-1,3-dithian
2-(4-Heptylphenyl)-5-(4-propylphenyl)-1,3-dithian

2-(4-Butyloxyphenyl)-5-(4-propylphenyl)-1,3-dithian
2-(4-Cyanophenyl)-5-(4-propylphenyl)-1,3-dithian
2-(4-Butoxyphenyl)-5-heptyl-1,3-dithian
2'-(4-Hexyloxyphenyl)-5-heptyl-1,3-dithian
2-(4-Heptyloxyphenyl)-5-heptyl-1,3-dithian
2'-(4-Octyloxyphenyl)-5-heptyl-1,3-dithian
2-(4-Butyloxyphenyl)-5-pentyl-1,3-dithian
2-(4-Fluorophenyl)-5-heptyl-1,3-dithian
2-(4-Cyanophenyl)-5-heptyl-1,3-dithian
2-[4-(4'-Ethoxybenzoyloxy)-phenyl]-5-butyl-1,3-dithian
2-[4-(4'-Butoxybenzoyloxy)-phenyl]-5-butyl-1,3-dithian
2-[4-(4'-Butyloxybenzoyloxy)-phenyl]-5-heptyl-1,3-dithian
2-[4-(4'-Fluorobenzoyloxy)-phenyl]-5-heptyl-1,3-dithian
2-[4-(4'-Cyanobenzoyloxy)-phenyl]-5-heptyl-1,3-dithian

bevorzugt

2-(4-Butoxyphenyl)-5-heptyl-1,3-dithian
2'-(4-Hexyloxyphenyl)-5-heptyl-1,3-dithian
2-(4-Heptyloxyphenyl)-5-heptyl-1,3-dithian
2'-(4-Octyloxyphenyl)-5-heptyl-1,3-dithian
2-(4-Butyloxyphenyl)-5-pentyl-1,3-dithian
2-(4-Fluorophenyl)-5-heptyl-1,3-dithian
2-(4-Cyanophenyl)-5-heptyl-1,3-dithian

besonders bevorzugt

2-[4-(4'-Ethoxybenzoyloxy)-phenyl]-5-butyl-1,3-dithian
2-[4-(4'-Butoxybenzoyloxy)-phenyl]-5-butyl-1,3-dithian
2-[4-(4'-Butyloxybenzoyloxy)-phenyl]-5-heptyl-1,3-dithian
2-[4-(4'-Fluorobenzoyloxy)-phenyl]-5-heptyl-1,3-dithian
2-[4-(4'-Cyanobenzoyloxy)-phenyl]-5-heptyl-1,3-dithian.

Die erfindungsgemäßen 2,5-disubstituierten 1,2-Dithiane können durch Umsetzung von Alkanalen oder para-substituierten Benzaldehyden der allgemeinen Formel

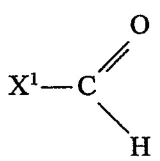

(IV)

in der

$X^1$ die vorgenannte Bedeutung hat und zusätzlich noch für eine p-Hydroxyphenylgruppe stehen kann,

mit 2-substituierten 1,3-Propandithiolen der allgemeinen Formel

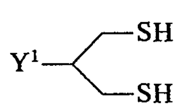

(V)

in der

$Y^1$ die vorgenannte Bedeutung hat und zusätzlich noch für eine p-Hydroxyphenylgruppe stehen kann,

in Gegenwart eines inerten, organischen Lösungs- und/oder Verdünnungsmittels und in Gegenwart eines sauren Katalysators hergestellt werden, wobei, wenn $X^1$ und/oder $Y^1$ eine p-Hydroxyphenylgruppe bedeuten, noch ein Veresterungs- oder Veretherungsschritt erfolgt.

Die erfindungsgemäße Umsetzung wird im allgemeinen bei Temperaturen von etwa 20 bis 80°C, bevorzugt bei 30 bis 50°C, durchgeführt.

Üblicherweise setzt man etwa 0,8 bis 1,3 Mol, bevorzugt 0,9 bis 1,2 Mol, besonders bevorzugt 1 Mol, Alkanale oder p-substituiertes Benzaldehyde der Formel (IV) pro Mol 1,3-Propandithiol der Formel (V) ein.

Als inerte, organische Lösungs- und/oder Verdünnungsmittel können Methylenchlorid, Chloroform,

Dichlorethan, Benzol und/oder Ether, wie Diethylether, bevorzugt Methylenchlorid und/oder Dichlorethan, eingesetzt werden.

Die Menge der eingesetzten Lösungs- und/oder Verdünnungsmittel kann in weiten Bereichen schwanken. Sie liegt im allgemeinen bei 1 bis 3 l, bevorzugt bei 2 l, bezogen auf 1 Mol eingesetztes 1,3-Propandithiol der Formel V.

Als saure Katalysatoren können sowohl Lewis- als auch Brönsted-Säuren, wie $ZnCl_2$, $AlCl_3$, $BF_3$-Etherat, $H_2SO_4$ und/oder HCl, bevorzugt $AlCl_3$ und/oder $BF_3$-Etherat, eingesetzt werden. Die eingesetzte Menge der sauren Katalysatoren kann ebenfalls in weiten Grenzen schwanken. Man setzt im allgemeinen 0,1 bis 10 Mol-%, bevorzugt 1 Mol-%, bezogen auf eingesetztes 1,3-Propandithiol, ein.

Wenn man p-Hydroxybenzaldehyd mit 2-Heptyl-1,3-propandithiol nach dem erfindungsgemäßen Verfahren umsetzt, so wird das daraus erhaltene 1,3-Dithian anschließend noch mit den entsprechenden Alkylhalogeniden oder Säurechloriden zu den 2,5-disubstituierten 1,3-Dithianen der Formeln (I) bis (III) verestert oder verethert.

Die Veresterung oder Veretherung der Phenolgruppe erfolgt dabei nach den bekannten Methoden, wie sie z. B. in Houben-Weyl, Bd. 8, S. 543ff. (1952) oder Houben-Weyl, Bd. 633, S. 49ff. (1965) beschrieben sind.

Das erfindungsgemäße Verfahren kann am Beispiel der Umsetzung von 2-Heptyl-1,3-propandithiol mit p-Hydroxybenzaldehyd und n-Butylbromid formelmäßig wie folgt dargestellt werden:

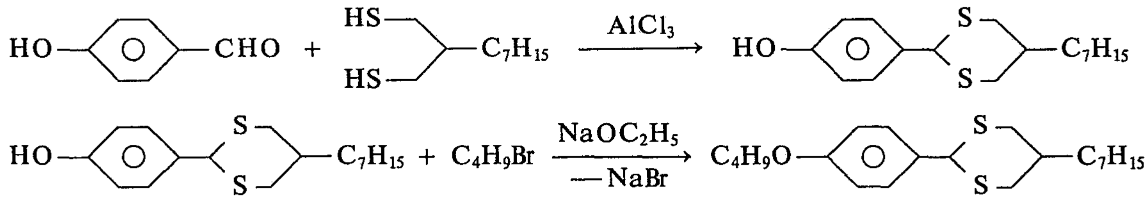


Die erfindungsgemäßen 2,5-disubstituierten-1,3-Dithiane besitzen anisotrope Eigenschaften, d. h. sie sind entweder enantiotrop flüssigkristallin oder monotrop flüssigkristallin, wobei bei monotrop flüssigkristallinen Verbindungen der Klärpunkt ($T_c$) der reinen Substanz niedriger liegt als deren Schmelzpunkt ($T_m$).

Die erfindungsgemäßen 2,5-disubstituierten-1,3-Dithiane können aufgrund ihrer anisotropen Eigenschaft als flüssigkristalline Verbindungen für die Konstruktion von optoelektronischen Bauelementen verwendet werden. Dabei werden die 1,3-Dithiane in Form ihrer Gemische untereinander oder mit anderen kristallinflüssigen Verbindungen, wie sie z. B. in der DE-OS 2 139 628 als p,p'-disubstituierte Benzoesäurephenylester und in der DE-OS 2 636 684 als p,p'-disubstituierte Phenylcyclohexane beschrieben sind, oder mit nicht-kristallinflüssigen Substanzen, z. B. mit Farbstoffen (beschrieben in Chimia 34 (2), 47 [1980]), eingesetzt. Wesentlich ist dabei, daß die eingesetzten Flüssigkristallmischungen mindestens eine der Verbindungen der Formel (I) enthalten. Die Verbindung der Formel (I) kann dann mit anderen Substanzen, wie anderen kristallinflüssigen Verbindungen oder nicht-kristallinflüssigen Verbindungen, in jedem beliebigen Mengenverhältnis in der Flüssigkristallmischung vorliegen. Im allgemeinen enthalten die erfindungsgemäßen Flüssigkristallmischungen 1 bis 95, bevorzugt 5 bis 50, Gew.-Teile der Verbindungen der Formel I.

Die Vorteile der erfindungsgemäßen 2,5-disubstituierten-1,3-Dithiane, die, wie oben dargelegt, in Form ihrer Gemische untereinander oder mit anderen kristallinflüssigen oder nicht-kristallinflüssigen Substanzen für optoelektronische Bauelemente verwendet werden können, bestehen in den meist niedrigen Schmelzpunkten bei hinreichend hohen Klärpunkten, in der Farblosigkeit und Beständigkeit unter Einwirkung von Licht, Chemikalien und thermischer Belastung.

Die folgenden Beispiele sollen die Herstellung der erfindungsgemäßen 2,5-disubstituierten-1,3-Dithiane verdeutlichen.

Beispiel 1

Darstellung von 2-substituierten 1,3-Propandithiolen

In einem 2-l-Dreihalskolben werden 2,1 Mol p-Toluolsulfonsäurechlorid in 1 l trockenem Pyridin vorgelegt und unter Kühlung und starkem Rühren 1 Mol 2-alkyl- bzw. 2-aryl-substituiertes 1,3-Propandiol (erhältlich durch $LiAlH_4$-Reduktion der entsprechenden 2-substituierten Malonsäureester analog E. L. Eliel und Sr. M. Carmeline Knoeber, H. Am. Chem. Soc. 90, 3444 [1968]) langsam zugesetzt. Nach Stehen über Nacht wird das Lösungsmittel abdestilliert und das in praktisch quantitativer Ausbeute erhaltene Ditosylat mit 5 Mol Thioharnstoff in 2 l Ethanol unter Stickstoff ca. 8 Stunden zum Rückfluß erhitzt. Anschließend wird das Ethanol abdestilliert und der Rückstand mit 380 g NaOH in 2 l Wasser weitere 8 h zum Rückfluß erhitzt. Danach wird angesäuert und mehrmals mit Chloroform extrahiert. Nach Trocknen der org. Phasen mit $Na_2SO_4$ wird das Lösungsmittel im Vakuum abdestilliert und der Rückstand fraktioniert bzw. umkristallisiert.

Beispiele sind in Tabelle 1 angegeben.

Tabelle 1: R $CH(CH_2SH)_2$

| R | Ausbeute (%) | Sdp. (°C/mm Hg) |
|---|---|---|
| n-$C_4H_9$ | 72 | 60—70/0,8 |
| n-$C_7H_{15}$ | 81 | 94—96/0,4 |
| $C_6H_5$ | 82 | 86—95/0,3 |
| $C_6H_5$—$CH_2$— | 86 | 102—107/0,8 |

## Beispiel 2

Darstellung von 2-(4-Hydroxyphenyl)-5-n-heptyl-1,3-dithian
(Formel I: X = p-HO—$C_6H_4$; Y = n-$C_7H_{15}$)

10,3 g (0,05 m) 2-n-Heptylpropandithiol-1,3 und 6,1 g (0,05 m) p-Hydroxybenzaldehyd werden in 100 ml Dichlorethan vorgelegt, eine katalytische Menge $AlCl_3$ zugegeben und über Nacht gerührt. Dann wird mehrmals mit Wasser ausgeschüttelt und anschließend die org. Phase über $Na_2SO_4$ getrocknet. Nach Abdestillieren des Lösungsmittels im Vakuum verbleiben 13,6 g (88%) farblose Kristalle, die durch Umkristallisation gereinigt werden. $T_m$ = 104—107°C; $T_c$ = /.

Nach der Arbeitsweise von Beispiel 2 werden unter Verwendung der entsprechenden 2-substituierten 1,3-Propandithiole und entsprechend substituierten Benzaldehyden die folgenden Verbindungen der Formel I hergestellt:

2-(4-Methoxyphenyl)-5-heptyl-1,3-dithian
2-(4-Nitrophenyl)-5-heptyl-1,3-dithian
2-(4-Dimethylamino)-5-heptyl-1,3-dithian
2-(4-Cyanophenyl)-5-heptyl-1,3-dithian
2-(4-Hydroxyphenyl)-5-heptyl-1,3-dithian
2-(4-Methoxyphenyl)-5-butyl-1,3-dithian
2-(4-Cyanophenyl)-5-butyl-1,3-dithian.

## Beispiel 3

Darstellung von 2-(4-n-Butyloxyphenyl)-5-n-heptyl-1,3-dithian
(Formel I: X = 4-$C_4H_9O$—$C_6H_4$; Y = $C_7H_{15}$)

6,2 g (0,02 m) 2-(4-Hydroxyphenyl)-5-heptyl-1,3-dithian werden unter Rühren in eine Lösung von 0,6 g (0,025 m) Natrium in 30 ml abs. Ethanol eingetragen. Danach werden 3,43 g (0,025 m) n-Butylbromid hinzugefügt und der Ansatz 5 Stunden zum Rückfluß erhitzt. Anschließend wird in Ether aufgenommen, mit Natronlauge und mit Wasser ausgeschüttelt. Nach Trocknen der org. Phase über $Na_2SO_4$ wird das Lösungsmittel im Vakuum abdestilliert. Zurück bleiben 6,5 g (89%) farblose Kristalle, die aus Ethanol umkristallisiert werden. $T_m$ = 58—59°C; $T_c$ = 39°C.

Nach der oben beschriebenen Arbeitsweise werden analog die folgenden Verbindungen der Formel I hergestellt:

2-(4-Hexyloxyphenyl)-5-heptyl-1,3-dithian
2-(4-Heptyloxyphenyl)-5-heptyl-1,3-dithian
2-(4-Octyloxyphenyl)-5-heptyl-1,3-dithian
2-(4-Butyloxyphenyl)-5-butyl-1,3-dithian
2-(4-Heptyloxyphenyl)-5-nutyl-1,3-dithian.

Beispiel 4

Darstellung von 2-[4-(4'-Butyloxybenzoyloxy)-phenyl]-5-butyl-1,3-dithian
(Formel I: X = $C_4H_9O-C_6H_4-COO-C_6H_4$; Y = $C_7H_{15}$)

In eine Lösung von 5,35 g (0,02 m) 2-(4-Hydroxyphenyl)-5-butyl-1,3-dithian in 30 ml Pyridin werden unter Rühren 6,39 g (0,03 m) 4-Butyloxybenzoylchlorid langsam eingetragen und über Nacht stehengelassen. Anschließend wird mit Ether aufgenommen und mehrmals mit Salzsäure ausgeschüttelt. Nach Trocknen der org. Phase mit $Na_2SO_4$ wird das Lösungsmittel im Vakuum abdestilliert.

Zurück bleiben 7,5 g (84%) farblose Kristalle, die aus Aceton umkristallisiert werden. $T_m$ 112–116°C; $T_c$ = 215°C.

Nach der oben beschriebenen Arbeitsweise werden analog die folgenden Verbindungen der Formel I hergestellt:

2-[4-(4'-Methoxybenzyloxy)-phenyl]-5-butyl-1,3-dithian
2-[4-(4'-Cyanobenzoyloxy)-phenyl]-5-butyl-1,3-dithian
2-[4-(4'-Butyloxybenzoyloxy)-phenyl]-5-heptyl-1,3-dithian
2-[4-(4'-Cyanobenzoyloxy)-phenyl]-5-heptyl-1,3-dithian
2-(4-Cyclohexylcarboxyphenyl)-5-heptyl-1,3-dithian.

**Patentansprüche**

1. 2,5-disubstituierte-1,3-Dithiane der Formel

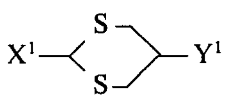

worin

$X^1$ = 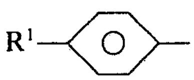 und $Y^1$ = $R^2$ oder

$X^1$ = $R^3$ und $Y^1$ = 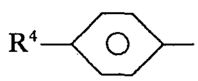 oder

$X^1$ = 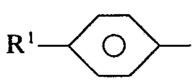 und $Y^1$ = 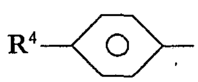

bedeuten und

$R^1$ = $C_nH_{2n+1}$, $C_nH_{2n+1}O$, $C_nH_{2n+1}S$, $C_nH_{2n+1}COO$, $C_nH_{2n+1}OCOO$, $(C_2H_5)_2N$, $(CH_3)_2N$, F, Cl, Br, $NO_2$, CN,

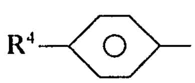 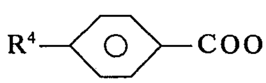 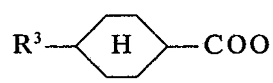

$R^2$ = $C_nH_{2n+1}$, $OR^3$, $SR^3$, $R^3COO$, $R^3OCO$, 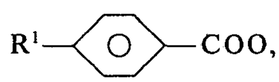, 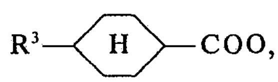, CN,

$R^3$ = $C_nH_{2n+1}$ und

$R^4$ = $C_nH_{2n+1}$, $C_nH_{2n+1}O$, F, Cl, Br, $NO_2$, CN

mit n = 1 bis 12 sein kann,
mit Ausnahme der 1,3-Dithiane der Formel

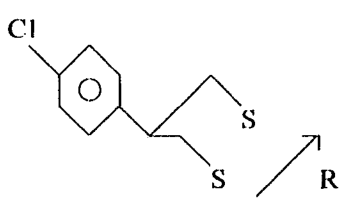

worin R für Methyl oder tertiär-Butyl steht.

2. Verfahren zur Herstellung von 2,5-disubstituierten-1,3-Dithianen der Formel

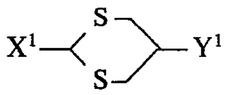

gemäß Anspruch 1, dadurch gekennzeichnet, daß man Alkanale oder para-substituierte Benzaldehyde der Formel

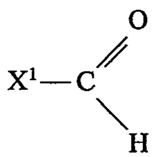

in der

$X^1$ die vorgenannte Bedeutung hat und zusätzlich noch für eine p-Hydroxyphenylgruppe stehen kann,

mit 2-substituierten 1,3-Propandithiolen der Formel

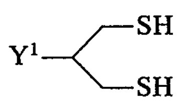

in der

$Y^1$ die vorgenannte Bedeutung hat und zusätzlich noch für eine p-Hydroxyphenylgruppe stehen kann,

in Gegenwart eines inerten, organischen Lösungs- und/oder Verdünnungsmittels und in Gegenwart eines sauren Katalysators umsetzt, wobei, wenn $X^1$ und/oder $Y^1$ für eine p-Hydroxyphenylgruppe stehen, sich noch ein Veresterungs- oder Veretherungsschritt anschließt.

3. Verfahren nach Anspruch 2, dadurch gekennzeichnet, daß man die Umsetzung bei Temperaturen von 20 bis 80°C durchführt.

4. Verfahren nach Anspruch 2, dadurch gekennzeichnet, daß man die Umsetzung bei 30 bis 50°C durchführt.

5. Verfahren nach Ansprüchen 2 bis 4, dadurch gekennzeichnet, daß man 0,8 bis 1,3 Mol Alkanale oder p-substituierte Benzaldehyde pro Mol 2-substituiertem 1,3-Propandithiol einsetzt.

6. Verfahren nach Ansprüchen 2 bis 4, dadurch gekennzeichnet, daß man 1 Mol Alkanale oder p-substituierte Benzaldehyde pro Mol 2-substituiertem 1,3-Propandithiol einsetzt.

7. Verfahren nach Ansprüchen 2 bis 6, dadurch gekennzeichnet, daß man als inerte, organische Lösungs- und/oder Verdünnungsmittel Methylenchlorid, Chloroform, Dichlorethan, Benzol und/oder Diethylether einsetzt.

8. Verfahren nach Ansprüchen 2 bis 7, dadurch gekennzeichnet, daß man als saure Katalysatoren Lewis- und/oder Brönsted-Säuren einsetzt.

9. Flüssigkristallmischungen enthaltend mindestens eine der Verbindungen der Formel

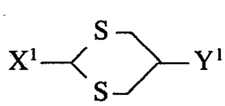

worin

$X^1$ = 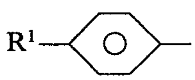 und $Y^1$ = $R^2$ oder

$X^1$ = $R^3$ und $Y^1$ = 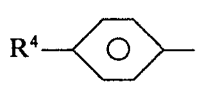 oder

$X^1$ = 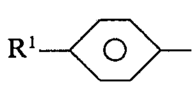 und $Y^1$ = 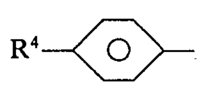

bedeuten und

$R^1$ = $C_nH_{2n+1}$, $C_nH_{2n+1}O$, $C_nH_{2n+1}S$, $C_nH_{2n+1}COO$, $C_nH_{2n+1}OCOO$, $(C_2H_5)_2N$, $(CH_3)_2N$, F, Cl, Br, $NO_2$, CN,

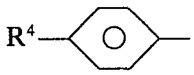 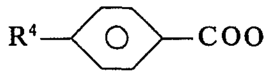 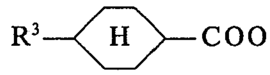

$R^2$ = $C_nH_{2n+1}$, $OR^3$, $SR^3$, $R^3COO$, $R^3OCO$, 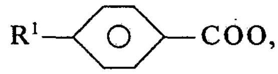, 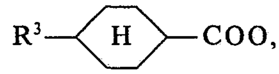, CN,

$R^3$ = $C_nH_{2n+1}$ und

$R^4$ = $C_nH_{2n+1}$, $C_nH_{2n+1}O$, F, Cl, Br, $NO_2$, CN

mit n = 1 bis 12 sein kann.

10. Verwendung der Flüssigkristallmischungen in optoelektronischen Bauelementen.

## Claims

1. 2,5-Disubstituted 1,3-dithianes of the formula

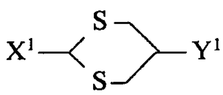

wherein

| | | | |
|---|---|---|---|
| $X^1$ denotes 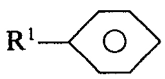 | and | $Y^1$ denotes $R^2$ | or |
| $X^1$ denotes $R^3$ | and | $Y^1$ denotes 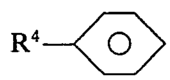 | or |
| $X^1$ denotes 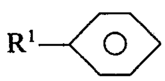 | and | $Y^1$ denotes 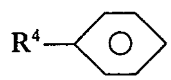 | |

and

$R^1$ can be $C_nH_{2n+1}$, $C_nH_{2n+1}O$, $C_nH_{2n+1}S$, $C_nH_{2n+1}COO$, $C_nH_{2n+1}OCOO$, $(C_2H_5)_2N$, $(CH_3)_2N$, F, Cl, Br, $NO_2$, CN,

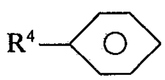 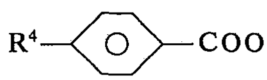 or 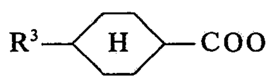

$R^2$ can be $C_nH_{2n+1}$, $OR^3$, $SR^3$, $R^3COO$, $R^3OCO$, 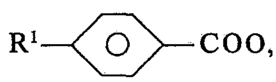, 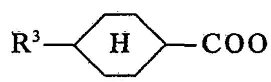 or CN,

$R^3$ can be $C_nH_{2n+1}$ and

$R^4$ can be $C_nH_{2n+1}$, $C_nH_{2n+1}O$, F, Cl, Br, $NO_2$ or CN,

in which n = 1 to 12,

with the exception of the 1,3-dithianes of the formula

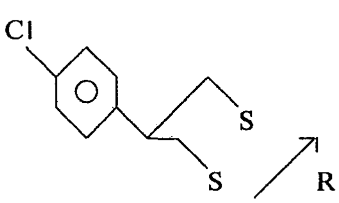

wherein R represents methyl or tertiary butyl.

2. Process for preparing 2,5-disubstituted 1,3-dithianes of the formula

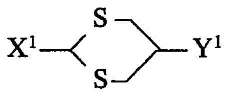

according to Claim 1, characterised in that alkanals or parasubstituted benzaldehydes of the formula

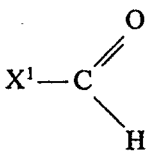

in which

$X^1$ has the abovementioned meaning and can additionally also represent a p-hydroxyphenyl group,

are reacted with 2-substituted 1,3-propanedithiols of the formula

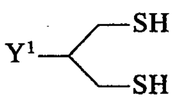

in which

$Y^1$ has the abovementioned meaning and can additionally also represent a p-hydroxyphenyl group,

in the presence of an inert, organic solvent and/or diluent and in the presence of an acid catalyst, followed, if $X^1$ and/or $Y^1$ represents a p-hydroxyphenyl group, by an esterifying or etherifying step.

3. Process according to Claim 2, characterised in that the reaction is carried out at temperatures of 20 to 80°C.

4. Process according to Claim 2, characterised in that the reaction is carried out at 30 to 50°C.

5. Process according to Claims 2 to 4, characterised in that 0.8 to 1.3 mols of alkanals or p-substituted benzaldehydes are used per mol of 2-substituted 1,3-propanedithiol.

6. Process according to Claims 2 to 4, characterised in that 1 mol of alkanals or p-substituted benzaldehydes is used per mol of 2-substituted 1,3-propanedithiol.

7. Process according to Claims 2 to 6, characterised in that the inert, organic solvents and/or diluents used are methylene chloride, chloroform, dichloroethane, benzene and/or diethyl ether.

8. Process according to Claims 2 to 7, characterised in that Lewis and/or Brønsted acids are used as acid catalysts.

9. Liquid crystal mixtures containing at least one of the compounds of the formula

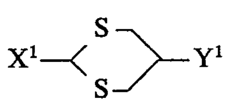

wherein

$X^1$ denotes 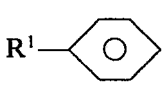 and $Y^1$ denotes $R^2$ or

$X^1$ denotes $R^3$ and $Y^1$ denotes 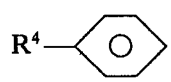 or

$X^1$ denotes 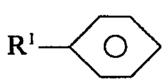 and $Y^1$ denotes 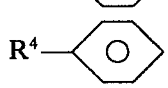

and

$R^1$ can be $C_nH_{2n+1}$, $C_nH_{2n+1}O$, $C_nH_{2n+1}S$, $C_nH_{2n+1}COO$, $C_nH_{2n+1}OCOO$, $(C_2H_5)_2N$, $(CH_3)_2N$, F, Cl, Br, $NO_2$, CN,

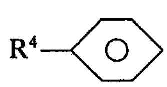 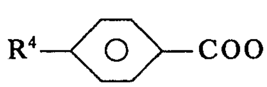 or 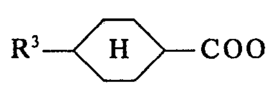

$R^2$ can be $C_nH_{2n+1}$, $OR^3$, $SR^3$, $R^3COO$, $R^3OCO$, $R^1$—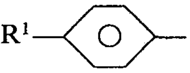—COO, $R^3$—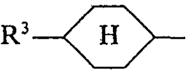—COO or CN,

$R^3$ can be $C_nH_{2n+1}$ and
$R^4$ can be $C_nH_{2n+1}$, $C_nH_{2n+1}O$, F, Cl, Br, $NO_2$ or CN,

in which n = 1 to 12.

10. Use of the liquid crystal mixtures in electro-optical components.

**Revendications**

1. 1,3-dithiannes 2,5-disubstitués de formule

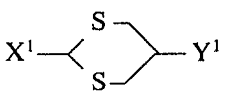

dans laquelle

$X^1$ = $R^1$—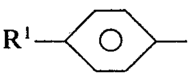— et $Y^1$ = $R^2$ ou

$X^1$ = $R^3$ et $Y^1$ = $R^4$—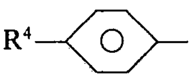— ou

$X^1$ = $R^1$—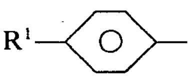— et $Y^1$ = $R^4$—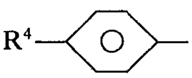—

et

$R^1$ = $C_nH_{2n+1}$, $C_nH_{2n+1}O$, $C_nH_{2n+1}S$, $C_nH_{2n+1}COO$, $C_nH_{2n+1}OCOO$, $(C_2H_5)_2N$, $(CH_3)_2N$, F, Cl, Br, $NO_2$, CN,

$R^4$—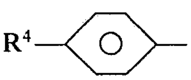— $R^4$—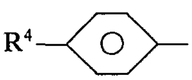—COO $R^3$—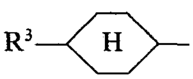—COO

$R^2$ = $C_nH_{2n+1}$, $OR^3$, $SR^3$, $R^3COO$, $R^3OCO$, $R^1$—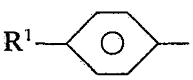—COO, $R^3$—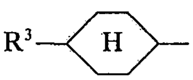—COO, CN,
$R^3$ = $C_nH_{2n+1}$ et
$R^4$ = $C_nH_{2n+1}$, $C_nH_{2n+1}O$, F, Cl, Br, $NO_2$, CN,

et n peut être un nombre de 1 à 12,

à l'exception des 1,3-dithiannes de formule

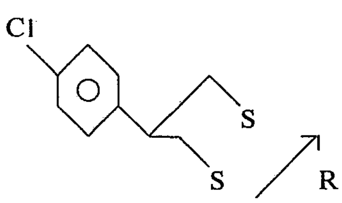

dans laquelle R représente un groupe méthyle ou tertiobutyle.

2. Procédé pour la fabrication de 1,3-dithiannes 2,5-disubstitués de formule

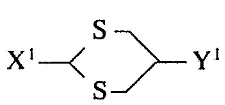

selon la revendication 1, caractérisé en ce que l'on fait réagir des alcanals ou des benzaldéhydes par a-substitués de formule

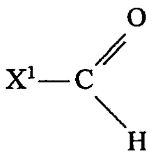

dans laquelle $X^1$ a la signification mentionnée ci-dessus et peut en outre représenter encore un groupe p-hydroxyphényle, avec des 1,3-propanedithiols 2-substitués de formule

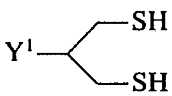

dans laquelle $Y^1$ a la signification mentionnée précédemment et peut encore représenter en outre un groupe p-hydroxyphényle, en présence d'un solvant et/ou diluant organique inerte et en présence d'un catalyseur acide, ladite réaction étant suivie encore d'une étape d'estérification ou d'éthérification dans le cas où $X^1$ et/ou $Y^1$ représentent un groupe p-hydroxyphényle.

3. Procédé selon la revendication 2, caractérisé en ce que l'on met en oeuvre la réaction à des températures de 20 à 80°C.

4. Procédé selon la revendication 2, caractérisé en ce que l'on met en oeuvre la réaction à 30—50°C.

5. Procédé selon les revendications 2 à 4, caractérisé en ce que l'on utilise 0,8 à 1,3 mole d'alcanals ou de benzaldéhydes p-substitués par mole de 1,3-propanedithiol 2-substitué.

6. Procédé selon les revendications 2 à 4, caractérisé en ce qu'on utilise 1 mole d'alcanals ou de benzaldéhydes p-substitués par mole de 1,3-popanedithiol 2-substitué.

7. Procédé selon les revendications 2 à 6, caractérisé en ce que l'on utilise comme solvants et/ou diluants organiques inertes le chlorure de méthylène, le chloroforme, le dichloroéthane, le benzène et/ou l'éther diéthylique.

8. Procédé selon les revendications 2 à 7, caractérisé en ce que l'on utilise comme catalyseurs acides des acides de Lewis et/ou des acides de Brönsted.

9. Mélanges de cristaux liquides contenant au moins l'un des composés de formule

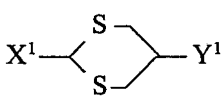

dans laquelle

$X^1$ = 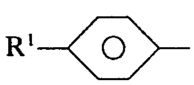 et $Y^1$ = $R^2$ ou

$X^1$ = $R^3$ et $Y^1$ = 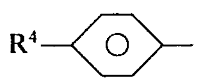 ou

$X^1$ = 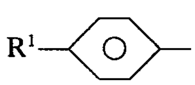 et $Y^1$ = 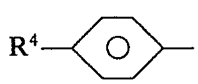

et

$R^1$ = $C_nH_{2n+1}$, $C_nH_{2n+1}O$, $C_nH_{2n+1}S$, $C_nH_{2n+1}COO$, $C_nH_{2n+1}OCOO$, $(C_2H_5)_2N$, $(CH_3)_2N$, F, Cl, Br, $NO_2$, CN,

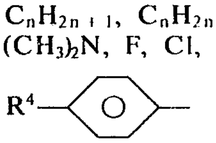 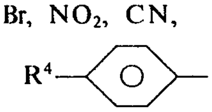COO 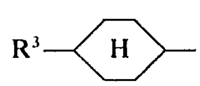COO

$R^2$ = $C_nH_{2n+1}$, $OR^3$, $SR^3$, $R^3COO$, $R^3OCO$, 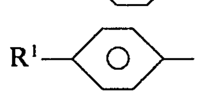COO, 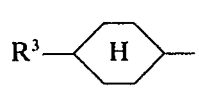COO, CN,

$R^3$ = $C_nH_{2n+1}$ et
$R^4$ = $C_nH_{2n+1}$, $C_nH_{2n+1}O$, F, Cl, Br, $NO_2$, CN,

et n peut être un nombre de 1 à 12.

10. Utilisation des mélanges de cristaux liquides dans des éléments de construction optoélectroniques.